# EUROPÄISCHE PATENTSCHRIFT

(11) **EP 1 273 269 B1**
(45) Veröffentlichungstag und Bekanntmachung des Hinweises auf die Patenterteilung: **03.01.2007**
(21) Anmeldenummer: 02007793.9
(22) Anmeldetag: 06.04.2002
(51) Int. Cl.: A61B 17/70, A61B 17/86, A61B 17/72

(54) **Implantierbare Schraube zur Stabilisierung einer Gelenkverbindung oder eines Knochenbruches**
Implantable screw for stabilising an articulated joint or a bone fracture
Vis implantable pour stabiliser un assemblage articulé ou une fracture d'os

(30) Priorität: 21.06.2001 DE 10129490
(43) Veröffentlichungstag der Anmeldung: 08.01.2003
(73) Patentinhaber: Mückter, Helmut, Dr. med. Dipl.-Ing., 52076 Aachen (DE); Hildinger, Karl Heinz, 52080 Aachen (DE)
(72) Erfinder: Mückter, Helmut, Dr. med. Dipl.-Ing., 52076 Aachen (DE)
(74) Vertreter: Castell, Klaus

(56) Entgegenhaltungen:
- EP-A- 0 374 088
- DE-U- 29 915 204
- FR-A- 2 784 019
- US-A- 4 959 064
- US-A- 5 735 898
- US-A- 5 814 047
- US-A- 6 010 507

## Beschreibung

Die Erfindung betrifft ein Implantat zur augmentierenden Stabilisierung von Knochengelenken mit geringer Relativbewegung und zur interfragmentären Stabilisierung von Knochenbrüchen, wenn vorwiegend Zugkräfte eingeleitet werden sollen.

Die Gelenke des Körpers weisen unterschiedliche Bewegungsausmaße auf. Neben den Gelenken in den Hauptbewegungsachsen der Extremitäten sowie dem Kiefergelenk, welche z.T. Relativbewegungen ganz erheblichen Ausmaßes aufweisen, existieren viele Gelenke mit geringem relativen Bewegungsausmaß. Typische Beispiele hierfür sind das Schultereckgelenk als Verbindung zwischen Schulterblatt und Schlüsselbein (Akromioldavikulargelenk), die Gelenkverbindung zwischen Schüsselbein und Brustbein (Sternoklavikulargelenk), das Kreuzdarmbeingelenk (Iliosakralgelenk), die Schambeinfuge (Symphyse), die gelenkigen Verbindungen zwischen Schien- und Wadenbein (proximales und distales Tibiofibulargelenk), die Gelenkverbindungen zwischen den Hand- und Fußwurzelknochen sowie die Gelenkverbindungen zwischen den Knochen der Mittelhand (Metakarpalgelenke) der des Mittelfußes (Metatarsalgelenke). Gleichwohl führen auch Verletzungen dieser Gelenkverbindungen in vielen Fällen zu schwerwiegenden körperlichen Einschränkungen, wobei sich als Folge einer verbleibenden Gelenkinkongruenz eine schmerzhafte Arthrose ausbildet. Therapeutisches Ziel muss daher die exakte Reposition dieser Gelenkverbindungen mit Wiederherstellung des Kapselbandapparates sein. Dies ist in der Regel durch eine einfache Naht des Kapsel-Bandapparates nicht erreichbar. Die Nähte würden der Belastung nicht standhalten, ausreißen und die Gelenkverbindung wieder in eine inkongruente Fehlstellung abgleiten. Vielmehr muss das verletzte Gelenk durch ein geeignetes chirurgisches Implantat im Sinne einer Augmentation solange in einer korrekten Stellung gehalten werden, bis der Kapsel-Bandapparat in ausreichender Festigkeit geheilt ist und die zur Führung der Gelenkverbindung erforderlichen Kräfte wieder übernehmen kann. Gleiches gilt für instabile Knochenbrüche, bei denen nach Reposition der Fraktur ein Implantat den Knochen solange in der korrekten Stellung halten soll, bis die Fraktur in ausreichender Festigkeit verheilt ist.

Zur augmentierenden Stabilisierung von zerrissenen Gelenkverbindungen mit geringen Relativbewegungen sind verschiedene Techniken beschrieben, welche grob in 4 Gruppen unterteilt werden können: 1. temporäre starre Überbrückung der Gelenkverbindung, 2. Überbrückung mit flexiblen Implantaten, 3. Halteimplantate, welche auf einer Gelenkseite verschraubt werden und auf der Gegenseite hakenförmig eingreifen, 4. Implantate mit gelenkiger Verbindung.

Bekanntester Vertreter der 1. Gruppe (starre Implantate) ist die sogenannte Stellschraube. Bei Anwendung dieses Prinzips werden beide Gelenkpartner durch eine direkte Verschraubung weitestgehend starr gegeneinander fixiert, wodurch zwar die Gelenkkongruenz gewährleistet wird, die Relativbewegung des Gelenkes aber blockiert wird. Ähnliche Funktionen werden durch Überbrückung der Gelenke mit Kirschnerdrähten, ggf. auch ergänzt durch Drahtcerclagen oder durch die Anwendung starrer Osteosyntheseplatten (insbesondere im Bereich des Beckens) erreicht.

Bekannte Vertreter der 2. Gruppe (flexible Implantate) sind Kunststoffkordeln oder -Bänder aus resorbierbarem oder nicht resorbierbarem Material (Literatur: Fremerey RW et al (1996) Die operative Behandlung der akuten, kompletten AC-Gelenkssprengung. Unfallchirurg 99:341-5), Drahtseile in der von LABITZKE (Literatur: Labitzke R (1982) Drahtseile und intraossäre Druckverteilungssysteme in der Chirurgie. Chirurg 53:741-3) vorgeschlagenen Technik oder die Anwendung von Drahtcerclagen.

Bekannte Vertreter der 3. Gruppe (verschraubte Implantate mit Haken) sind die von BALSER, WOLTER oder von DREITHALER in ähnlicher Bauweise vorgeschlagenen Hakenplatten zur Stabilisierung von Sprengungen des Schultereckgelenkes oder der von ENGELBRECHT (Literatur: Engelbrecht E et al. (1971) Die Versorgung tibio-fibularer Syndesmosensprengungen mit dem Syndesmosenhaken. Chirurg 42:92) entwickelte Syndesmosenhaken zur Stabilisierung von Sprengungen der Knöchelgabel. Diese Implantate erlauben eine gute Augmentation der Gelenkverbindung und erhalten im wesentlichen die Beweglichkeit, die Einstellung der korrekten Gelenkkongruenz ist jedoch schwierig und kann mitunter nur durch Nachbiegen des Implantates erreicht werden, da diese Implantate keine geeigneten Verstellmöglichkeiten aufweisen. Außerdem ist ein relativ gro-ßer operativer Zugang erforderlich, welcher eine Erweiterung des Operationstraumas bedingt.

Ein typischer Vertreter der 4. Gruppe (Implantate mit integrierter Gelenkverbindung) ist die von RAMANZADEH entwickelte Gelenkplatte zur Stabilisierung von Sprengungen des Schultereckgelenkes. Diese Platte besitzt jedoch den Nachteil, dass die Einstellung einer korrekten Kongruenz des Gelenkes schwierig ist, und dass die Drehachsen des Gelenkes und des Implantates nicht übereinstimmen, wodurch die natürliche Gelenkbewegung zumindest teilweise blockiert wird

Die Entgegenhaltung DE 299 15 204 und die Patentschrift US 4,959,064 beschreiben eine Schraube, die im Schaftbereich eine Spiralfeder aufweist. Eine Spiralfeder führt zu einem flexiblen Schraubenschaft, welcher sich unter Zugkraft in Längsrichtung dehnt. Bei der Verwendung einer derartigen Schraube zur Behandlung einer Sprunggelenksgabel mit zerrissenem Syndesmosenband weicht die Knöchelgabel auseinander, woraus eine Verschiebung des Sprungbeins in der Knöchelgabel und damit eine Inkongruenz des oberen Sprunggelenks resultiert. Hierbei droht die Gefahr eines schwerwiegenden Gelenkschadens mit resultierenden Knorpelschäden und der Gefahr einer Arthrose.

Der Erfindung liegt daher die Aufgabe zugrunde, ein Implantat zu entwickeln, welches die Bandverbindungen bei Gelenken mit geringer Relativbewegung zuverlässig augmentiert, dabei aber das natürliche Bewegungsausmaß des Gelenkes nicht oder nicht wesentlich behindert.

Diese Aufgabe wird mit einer implantierbaren Schraube zur Stabilisierung einer Gelenkverbindung oder eines Knochenbruches mit flexiblem Schaft gelöst, bei der die Flexibilität im Schaftbereich durch ein Drahtseil, eine Kordel, ein Drahtbündel oder durch Fäden erzielt wird.

Diese Bauweise des Implantates gewährleistet fast ausschließlich eine Übertragung von Zugkräften, während Biegemomente, Druck - und Querkräfte durch den flexiblen Schaft nicht bzw. nur unwesentlich übertragen werden.

Ein Drahtseil, eine Kordel, ein Drahtbündel oder Fäden können im Hinblick auf ihre Funktion als Seile bezeichnet werden. Während sich eine Schraube mit Spiralfeder im Schaftbereich unter einer Zugkraft in Längsrichtung dehnt, kommt es bei einem Seil im Schaftbereich auch unter relativ hohen einwirkenden Kräften nicht zu einem Auseinanderweichen der Knöchelgabel. Dagegen lassen sich alle anderen Relativbewegungen (Drehung des Außenknöchels und leichte Translationsbewegungen zwischen Schien- und Wadenbein) ohne relevanten Kraftaufwand ausführen. Durch die Verwendung eines Seiles lässt sich daher die stabilisierende Funktion einer natürlichen Bandverbindung in annährend physiologischer Weise ersetzen. Die Anwendung einer oder mehrerer derartiger Schrauben mit flexiblem Schaft ermöglicht es, die an einer instabilen Gelenkverbindung beteiligten Knochen so miteinander zu verbinden, dass die natürliche Gelenkbewegung nicht oder nur unwesentlich behindert wird.

Ebenso wird es durch die Anwendung einer oder mehrerer derartiger Schrauben mit flexiblem Schaft ermöglicht, bei einer Verschraubung von Knochenbrüchen vorwiegend interfragmentäre Zugkräfte einzuleiten.

Eine derartige Schraube ermöglicht es, dass beide an einer Gelenkverletzung beteiligten Knochen oder an einer Knochenverletzung beteiligten Fragmente durch eine oder mehrere Schrauben verbunden werden, welche einen flexiblen Schaft aufweisen. Diese Bauweise des Implantates gewährleistet fast ausschließlich eine Übertragung von Zugkräften, während Biegemomente, Druck- und Querkräfte durch den flexiblen Schaft nicht bzw. nur unwesentlich übertragen werden. Bei Kapselbandverletzungen eines Gelenkes wird die Schraube bevorzugt so montiert, dass die Schraubenachse mit der Richtung der resultierenden Kraft der Bandverbindung des Gelenkes übereinstimmt. Dabei lässt sich eine ideale Augmentation der Gelenkverbindung erreichen. Bei Knochenbrüchen wird die Schraube senkrecht zur Frakturebene eingebracht und bewirkt durch die Zugkraft eine interfragmentäre Kompression.

Vorteilhafterweise kann durch die Erfindung eine Ausweitung des Operationsraumes begrenzt werden. Die Erfindung ist in vorteilhafter Ausgestaltung zur sogenannten minimal-invasiven Implantation geeignet.

Darüber hinaus kann die erfindungsgemäße Schraube zur Verwendung in der Chirurgie so gestaltet werden, dass vorwiegend Zugkräfte und keine wesentlichen Biegemomente übertragen werden. Ebenso kann die erfindungsgemäße Schraube so ausgebildet werden, dass diese im Sinne einer sogenannten Kriechschraube in die Markhöhle eines frakturierten Knochens eingebracht werden kann, und sich dabei der meist gebogenen Kontur der Markhöhle anpasst.

Vorteilhaft ist es, wenn das axiale Flächenträgheitsmoment der Schraube um 30%, vorzugsweise mehr als 50% kleiner ist als bei einer Schraube gleichen äußeren Durchmessers.

Die Flexibilität wird im Schaftbereich durch ein Drahtseil, ein Drahtbündel, eine Kordel oder durch Fäden erzielt.

Bei Verwendung eines Drahtseiles oder eines Drahtbündels ist es besonders vorteilhaft, wenn das Drahtseil oder das Drahtbündel von außen durch Hülsen oder eine Spirale armiert ist. Bei der Einleitung von Torsionsmomenten wird dadurch ein Aufwickeln des Drahtseiles oder der Drahtbündel begrenzt und hierdurch das Drahtseil bzw. des Drahtbündel stabilisiert. Außerdem lässt sich abhängig von der Größe der Hülsen bzw. der Spiralwindungen und deren Abstand zueinander die Biegebewegung des Schaftes begrenzen.

Besonders vorteilhaft ist es, wenn der Gewindeteil ein Knochengewinde aufweist.

Ein bevorzugtes Ausführungsbeispiel sieht vor, dass der Kopfteil eine Schraubenschlüsselaufnahme aufweist und je nach Einsatzzweck des Implantates eine glatte Oberfläche oder ein Knochengewinde entsprechend dem Gewindeteil aufweist oder ein Knochengewinde aufweist, welches im Durchmesser größer und in der Gewindesteigung kleiner ist als das Knochengewinde im Gewindeteil.

Bei hoher Flexibilität des Schaftes und dadurch bedingter unzureichender Übertragbarkeit der zum Eindringen des Gewindes erforderlichen Torsionsmomente ist es von Vorteil, wenn die implantierbare Schraube Schraubenschlüsselaufnahmen sowohl im Kopfteil als auch im Gewindeteil aufweist. Dies ermöglicht es, einen stufenförmigen Schraubenschlüssel synchron an diesen Schraubenschlüsselaufnahmen angreifen zu lassen.

Weitere vorteilhafte Ausführungsvarianten sind in den Unteransprüchen beschrieben.

Verschiedene Ausführungsbeispiele der Erfindung sind zeichnerisch dargestellt und werden im folgenden näher beschrieben.

Es zeigt
- Fig. 1: eine Knochenschraube, deren Schaft flexibel als Drahtseil oder als Drahtbündel ausgeführt ist,
- Fig. 2: eine Knochen-Stellschraube, deren Schaft flexibel als Drahtseil oder als Drahtbündel ausgeführt ist,
- Fig. 3: eine Knochenschraube, die auf der kopffernen Seite ein Knochengewinde aufweist, deren Schaft flexibel als Drahtseil oder als Drahtbündel ausgeführt ist und auf der Kopfseite ein Knochengewinde aufweist, das im Durchmesser größer ist und eine geringere Gewindesteigung aufweist als das kopfferne Knochengewinde,
- Fig. 4: eine Schraube, die auf der einen Seite ein Knochengewinde aufweist, deren Schaft flexibel als Drahtseil, als Kordel oder als Drahtbündel ausgeführt ist und auf der anderen Seite einen Bolzen mit Metallgewinde und aufgeschraubter Innensechskantkopfmutter aufweist,
- Fig. 5: einen stufenförmigen Innensechskantschlüssel,
- Fig. 6: eine Knochenschraube, bei welcher der Schaft aus multiplen Fäden besteht, die jeweils alternierend im Kopfteil und im Gewindeteil der Knochenschraube verankert sind,
- Fig. 7: ein Anwendungsbeispiel zur Stabilisierung der Knöchelgabel (distales Tibiofibulargelenk, Syndesmose)
- Fig. 8: ein Anwendungsbeispiel zur Stabilisierung des Schultereckgelenkes (Akromioklavikulargelenk)
- Fig. 9: ein Anwendungsbeispiel zur Stabilisierung des Kreuzdarmbeingelenkes (Iliosakralfuge),
- Fig. 10: ein Anwendungsbeispiel zur Stabilisierung im Bereich der Handwurzel bei skapholunärer Dissoziation,
- Fig. 11: ein Anwendungsbeispiel zur interfragmentären Zugverschraubung im Bereich der Kniescheibe bei einer Patellafraktur und
- Fig. 12: eine Knochenschraube entsprechend Figur 4, bei der das Drahtseil oder das Drahtbündel durch einzelne Hülsen armiert ist.

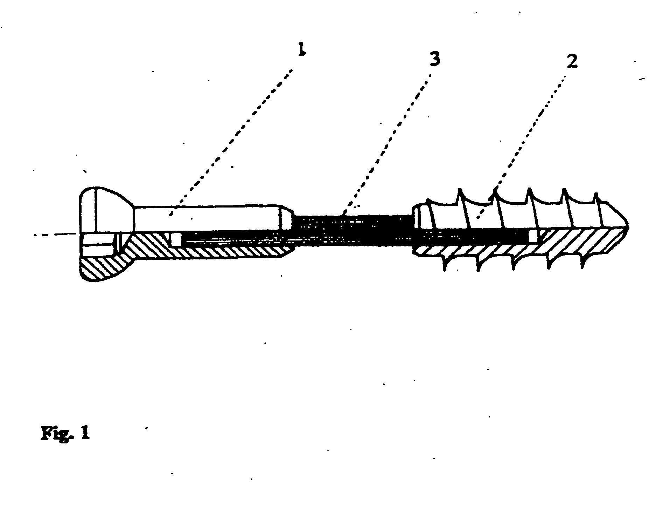

Die Fig. 1 zeigt eine Knochenschraube, deren Kopfteil 1 und deren Gewindeteil 2 durch ein Drahtseil oder ein Drahtbündel 3 flexibel miteinander verbunden sind. Das Drahtseil oder das Drahtbündel sind sowohl im Kopfteil, wie im Gewindeteil durch geeignete Verbindungsverfahren (z.B. Press-, Klebe-, Löt- oder Schweißverbindung) fest konnektiert. Die Verwendung eines Drahtseiles oder eines Drahtbündels gestattet die Einleitung von Zugkräften und die Übertragung von Torsionsmomenten im Sinne einer biegsamen Welle. Druckkräfte, Querkräfte oder Biegemomente werden dagegen nur in geringem Maße übertragen.

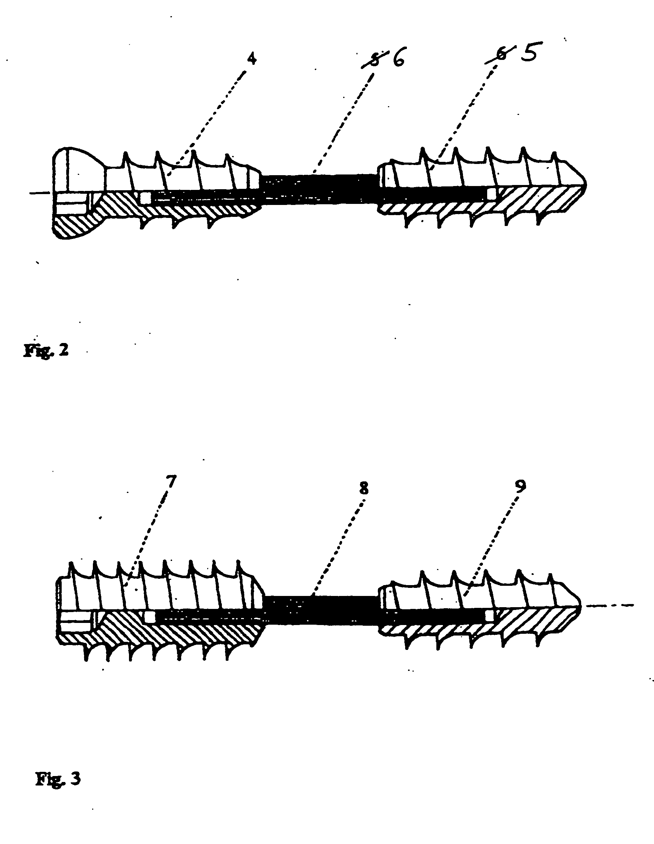

Die Fig. 2 zeigt eine Knochen-Stellschraube, die einen mit Knochengewinde versehenen Kopfteil 4 und einen Gewindeteil 5 aufweist, welche analog zu Fig. 1 durch ein Drahtseil oder ein Drahtbündel 6 flexibel miteinander verbunden sind. Dabei sind die Gewinde von Kopfteil und Gewindeteil von gleicher Größe und die Gewindeflanken deckungsgleich. Auf diese Weise wird ein zuvor definierter Abstand zwischen 2 zu verbindenden Knochen unabhängig vom Anzugsmoment der Schraube fixiert.

Die Fig. 3 zeigt eine Knochenschraube mit gewindetragendem Kopfteil 7, welcher über ein Drahtseil oder ein Drahtbündel 8 flexibel analog zu Fig. 1 mit dem Gewindeteil 9 verbunden sind. Entsprechend dem bekannten Funktionsprinzip der HERBERT-Schraube weist das Gewinde am Kopfteil gegenüber dem Gewindeteil einen größeren Durchmesser und eine kleinere Gewindesteigung auf. Beim Eindrehen dieser Schraube in einen frakturierten Knochen senkrecht zur Frakturebene werden die beiden Fragmente aufeinander zu bewegt und gegeneinander verspannt, wobei sich das Maß der Zueinanderbewegung pro Schraubenumdrehung aus der Differenz der beiden Gewindesteigungen ergibt.

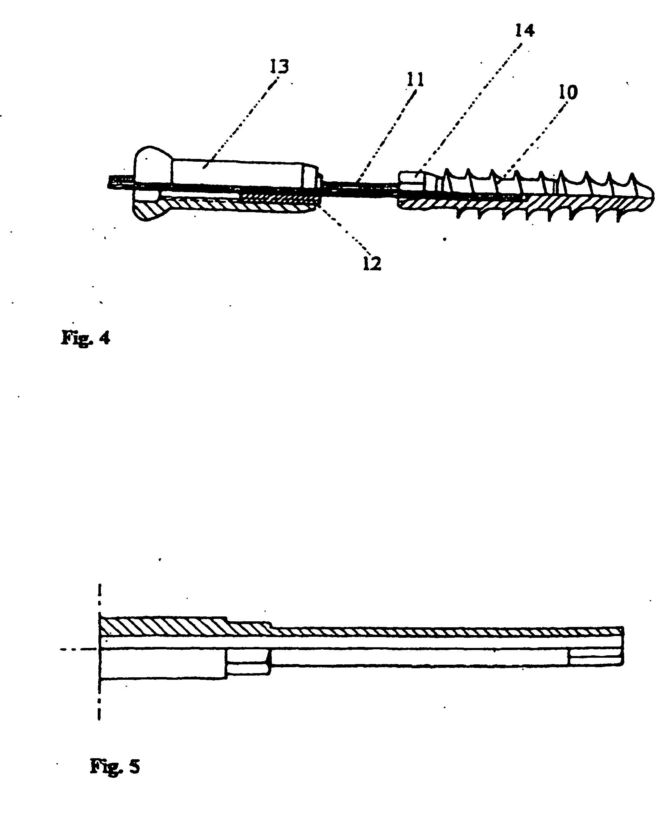

Die Fig. 4 zeigt eine Schraube, die auf der einen Seite einen Gewindeteil 10 mit Knochengewinde aufweist, welcher flexibel durch ein Drahtseil, ein Drahtbündel oder eine Kordel 11 mit einem Bolzen 12 verbunden ist, der ein Metall- oder Kunststoffgewinde aufweist. Auf diesen Bolzen ist eine Innensechskantkopfmutter 13 aufgeschraubt. Bei der Implantation einer solchen Schraube wird zunächst der Gewindeteil mit Knochengewinde im Sinne eines Stehbolzens in den Knochen eingeschraubt. Dies geschieht mittels eines kanülierten Steckschlüssels, welcher über das Drahtseil oder das Drahtbündel bzw. die Kordel und den Bolzen geschoben wird und am Außensechskant 14 des Gewindeteils eingreift. Danach wird die Innensechskantkopfmutter mittels eines kanülierten Innensechskantschlüssels auf den Bolzen mit Metallgewinde aufgeschraubt. Abschließend wird das am Innensechskant überstehende Drahtseil oder Drahtbündel bzw. die überstehende Kordel mit einer Kneifzange gekürzt.

Die Fig. 5 zeigt einen stufenförmigen Innensechskantschlüssel. Für manche Anwendungen ist es vorteilhaft, wenn der Gewindeteil kanüliert ist so dass die Applikation der Schraube über einen entsprechenden Führungsdraht erfolgen kann.

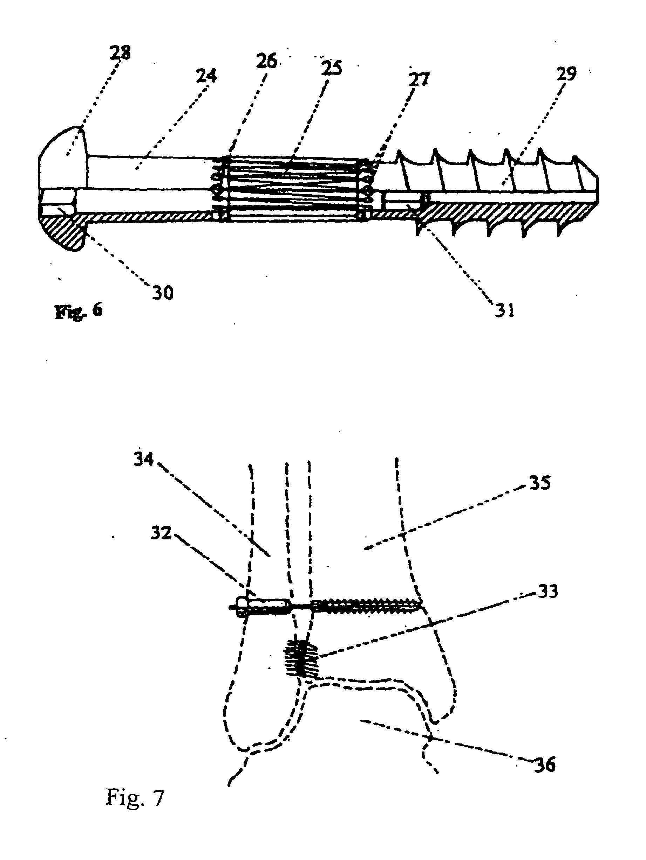

Die Fig. 6 zeigt eine Knochenschraube 24, welche sich gleichermaßen für die Fertigung aus Implantatmetall wie aus resorbierbaren oder nicht resorbierbaren Kunststoffen eignet, und welche so gestaltet ist, dass die einzelnen Komponenten gießtechnisch hergestellt werden können. Dabei wird die Flexibilität des Schaftes dadurch erreicht, dass dieser aus multiplen Fäden 25 besteht, welche entweder entsprechend der Abbildung in Ösen 26, 27 alternierend im Kopfteil 28 und im Gewindeteil 29 der Schraube gehalten werden oder jeweils im Kopfteil und im Gewindeteil fest verankert sind.

Die Fig. 7 zeigt ein Anwendungsbeispiel einer Knochenschraube mit flexiblem Schaft 32 nach Fig. 4, welche im Bereich der Knöchelgabel zur Augmentation einer zerrissenen Syndesmose 33 (Syndesmose = Bandverbindung zwischen dem Wadenbein 34 und dem Schienbein 35 im Bereich des Sprunggelenkes) eingebracht ist. Im Gegensatz zu einer konventionellen starren Verschraubung bleibt durch den flexiblen Schaft die natürliche Relativbewegung zwischen Wadenbein und Schienbein erhalten. Ein Auseinanderweichen der Knöchelgabel, welches zu einer Instabilität des Sprungbeines 36 führen würde, ist jedoch nicht möglich. Die Dimensionierung der Knochenschraube ist dabei so gewählt, dass sie beim Vorliegen einer begleitenden Außenknöchelfraktur durch die Bohrungen einer üblichen Osteosyntheseplatte in den Knochen eingedreht werden kann.

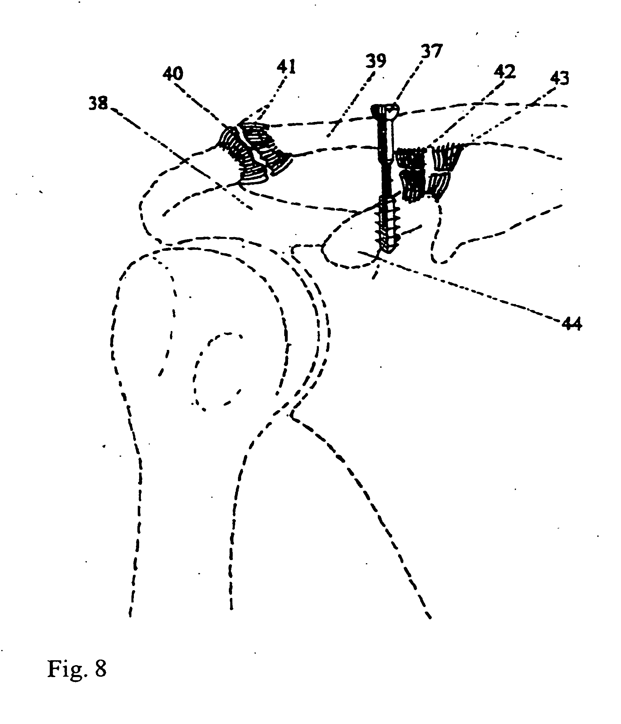

Die Fig. 8 zeigt ein weiteres Anwendungsbeispiel einer Knochenschraube mit flexiblem Schaft 37 nach Fig. 1 im Bereich der Bandverbindung zwischen Schulterblatt 38 und Schlüsselbein 39, am Schultereckgelenk 40 (Akromio-Klavikular-Gelenk). Es ist schematisch die Ruptur aller 3 an dieser Verbindung beteiligten Bänder (Lig. acromioclaviculare 41, Lig. trapezoideum 42, Lig. conoideum 43) eingezeichnet. Entsprechend dem 1941 von BOSWORTH für die Verwendung von starren Schrauben beschriebenen Prinzip ist die Schraube mit flexiblem Schaft durch das Schlüsselbein in den Rabenschnabel-Fortsatz 44 eingeschraubt. Im Gegensatz zu einer konventionellen starren Verschraubung bleibt durch den flexiblen Schaft die natürliche Relativbewegung zwischen Schlüsselbein und Schulterblatt erhalten. Ein Hochstand des Schlüsselbeines, welcher zu einer Inkongruenz des Schultereckgelenkes führen würde, ist jedoch nicht möglich.

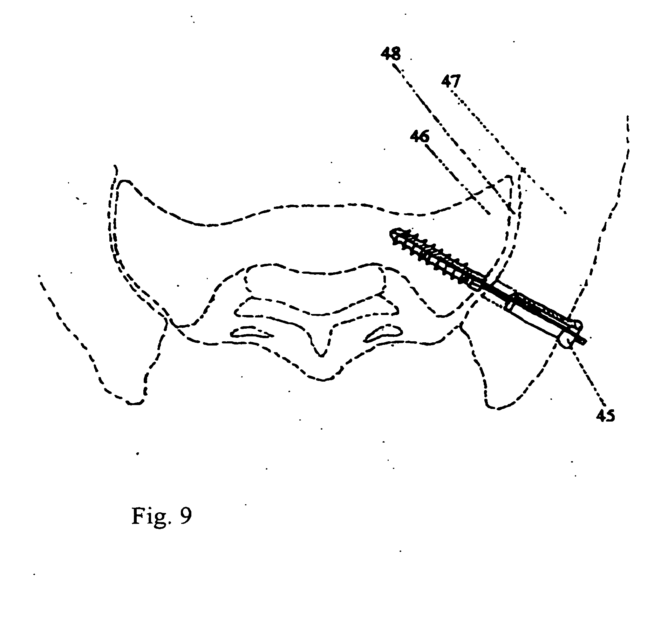

Die Fig. 9 zeigt ein weiteres Anwendungsbeispiel einer Knochenschraube mit flexiblem Schaft 45 nach Fig. 4 im Bereich der Bandverbindung zwischen dem Kreuzbein 46 und dem Darmbein 47 (Iliosakralgelenk 48). Bei einer verletzungsbedingten Instabilität des hinteren Beckenringes erfolgt die Stabilisierung durch Eindrehen einer oder mehrerer Schrauben mit flexiblem Schaft. Im Gegensatz zu einer konventionellen starren Verschraubung bleibt durch den flexiblen Schaft die natürliche Relativbewegung zwischen Kreuzbein und Darmbein erhalten. Ein Klaffen des Gelenkspaltes wird durch die Schraube mit flexiblem Schaft jedoch zuverlässig verhindert.

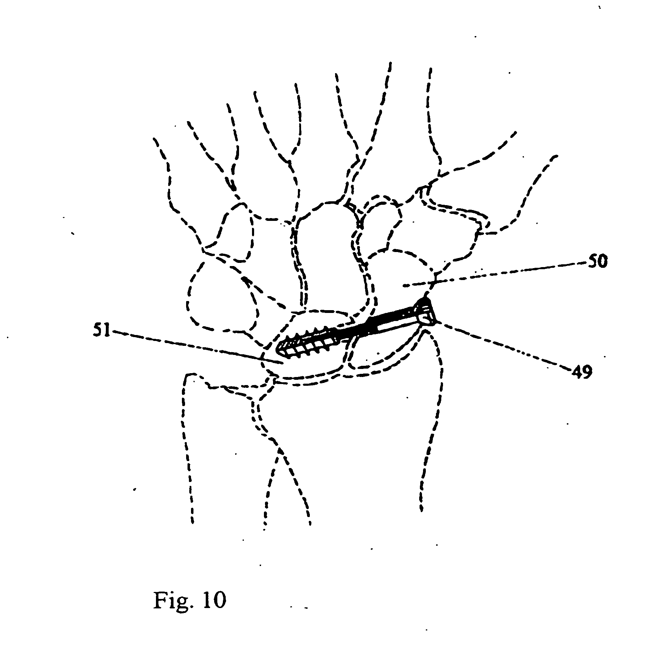

Die Fig. 10 zeigt ein weiteres Anwendungsbeispiel einer Knochenschraube mit flexiblem Schaft 49 nach Fig. 4 im Bereich der Handwurzel bei einer Bandzerreißung zwischen dem Kahnbein 50 und dem Mondbein 51 (Skapholunäre Dissoziation). Die Reposition und Stabilisierung erfolgt durch Eindrehen einer Schraube mit flexiblem Schaft. Im Gegensatz zu einer konventionellen starren Verschraubung oder Stabilisierung mit Kirschnerdrähten bleibt durch den flexiblen Schaft die natürliche Relativbewegung zwischen Kahnbein und Mondbein erhalten. Die miteinander verschraubten Handwurzelknochen können jedoch nicht auseinanderweichen.

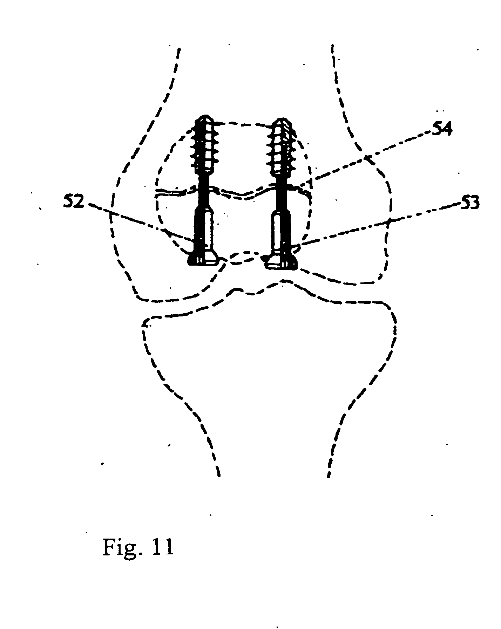

Die Fig. 11 zeigt ein weiteres Anwendungsbeispiel von Knochenschrauben mit flexiblem Schaft 52, 53 nach Fig. 1 bei einer Querfraktur der Patella 54. Entsprechend dem bekannten Zuggurtungsprinzip werden die von der Quadricepssehne über die Patella in die Patellasehne fortgeleiteten Zugkräfte durch die beiden Knochenschrauben mit flexiblem Schaft übertragen und die beiden Fragmente der Patella gegeneinander komprimiert.

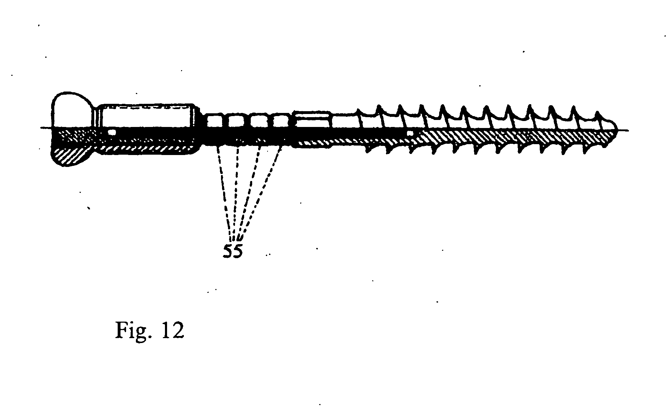

Die Fig. 12 zeigt eine Knochenschraube gemäß Fig. 4, bei der das Drahtseil oder das Drahtbündel durch einzelne Hülsen 55 armiert ist. Drahtseile neigen entsprechend ihrer Wickelung bei Einleitung eines Torsionsmomentes in Gegenrichtung dazu, sich aufzuwickeln. Durch das Aufschieben von Hülsen oder einer Spirale über das Drahtseil oder Drahtbündel lässt sich dieses Aufwickeln begrenzen und gleichzeitig durch die dabei auftretende Verklemmung des Drahtseiles in der Hülse oder der Spirale eine Stabilisierung des Drahtseiles erreichen. Hierdurch lassen sich höhere Torsionsmomente als mit einem nicht armierten Drahtseil oder Drahtbündel übertragen. Des weiteren lässt sich je nach Gestaltung der Hülsen und je nach Abstand der einzelnen Hülsen bzw. Spiralwindungen zueinander das Ausmaß der Biegung des flexiblen Schraubenschaftes begrenzen.

## Patentansprüche

1. Implantierbare Schraube zur Stabilisierung einer Gelenkverbindung oder eines Knochenbruches mit einem Kopfteil (1, 4, 7, 13, 28), einem Gewindeteil (2, 5, 9, 10,29), und einem flexiblen Schaft, ***dadurch gekennzeichnet, dass*** die Flexibilität im Schaftbereich durch ein Drahtseil (3), eine Kordel (11), ein Drahtbündel (6, 8) oder durch Fäden (25) erzielt wird.

2. Implantierbare Schraube nach Anspruch 1, ***dadurch gekennzeichnet, dass*** das im Schaftbereich angeordnete Drahtseil (3) oder Drahtbündel (6, 8) durch von außen über geschobene Hülsen (55) oder eine Spirale armiert ist.

3. Implantierbare Schraube nach einem der vorhergehenden Ansprüche, ***dadurch gekennzeichnet, dass*** ein Gewindeteil (5, 9, 10) als Stehbolzen mit einer Schraubenschlüsselaufnahme ausgeführt ist und im Bereich eines Kopfteiles (4) einen Gewindebolzen mit Kopfmutter aufweist.

4. Implantierbare Schraube nach Anspruch 3, ***dadurch gekennzeichnet, dass*** diese Schraubenschlüsselaufnahmen sowohl im Kopfteil (4) als auch im Gewindeteil (5, 9, 10) aufweist

5. Implantierbare Schraube nach einem der vorhergehenden Ansprüche, ***dadurch gekennzeichnet, dass*** ein Gewindeteil (5, 9, 10) ein Knochengewinde aufweist.

6. Implantierbare Schraube nach einem der vorhergehenden Ansprüche, ***dadurch gekennzeichnet* dass** axiale Flächenträgheitsmoment der Schraube um 30%, vorzugsweise 50% kleiner ist als bei einer Schraube gleichen äußeren Durchmessers.

7. Implantierbare Schraube nach einem der vorhergehenden Ansprüche, ***dadurch gekennzeichnet, dass*** als Werkstoff sowohl biokompatibles Metall als auch biokompatibler resorbierbarer oder nichtresorbierbarer Kunststoff oder eine Kombination aus solchen Materialien verwendet werden.

## Claims

1. An implantable screw for stabilising a joint or a bone fracture with a head part (1, 4, 7, 13, 28), a threaded part (2, 5, 9, 10, 29) and a flexible shaft, ***characterized in that*** the flexibility in the area of the shaft is achieved through a wire rope (3), a string (11), a bundle of wire (6, 8) or through threads (25).

2. The implantable screw according to claim 1, ***characterized in that*** the wire rope (3) or the bundle of wire (6, 8) disposed in the area of the shaft is reinforced by a sheath or a coil (55) pulled over from outside.

3. The implantable screw according to one of the afore-mentioned claims, ***characterized in that*** a threaded part (5, 9, 10) is configured as a stud bolt with a wrench receptacle and has a threaded bolt with a head nut in the area of a head part (4).

4. The implantable screw according to claim 3, ***characterized in that*** it has wrench receptacle as well in the head part (4) as in the threaded part (5, 9, 10).

5. The implantable screw according to one of the afore-mentioned claims, ***characterized in that*** a threaded part (5, 9, 10) has a bone thread.

6. The implantable screw according to one of the afore-mentioned claims, ***characterized in that*** the axial planar moment of inertia of the screw is 30%, preferably 50% smaller than with a screw having the same outer diameter.

7. The implantable screw according to one of the afore-mentioned claims, ***characterized in that*** as well biocompatible metal as absorbable or non absorbable biocompatible plastic or a combination of such materials are used as a material.

## Revendications

1. Vis implantable pour stabiliser une articulation ou une fracture osseuse avec une tête (1, 4, 7, 13, 28), une partie filetée (2, 5, 9, 10, 29), et avec une tige flexible, ***caractérisée en ce que*** la flexibilité de la zone de la tige est obtenue au moyen d'un câble métallique (3), d'un cordon (11), d'un faisceau de fil métallique (6, 8) ou au moyen de fils (25).

2. Vis implantable selon la revendication 1, ***caractérisée en ce que*** le câble métallique (3) ou le faisceau de fil métallique disposé dans la zone de la tige est renforcé par des gaines (55) ou une spirale.

3. Vis implantable selon l'une quelconque des revendications précédentes, ***caractérisée en ce qu*'**une partie filetée (5, 9, 10) est réalisée sous forme de goujon avec un logement pour une clé et qu'elle comporte dans la zone de la tête un goujon fileté avec un écrou de tête.

4. Vis implantable selon la revendication 3, ***caractérisée en ce que*** celle-ci comporte des logements pour clé sur la tête (4) ainsi que sur la partie filetée (5, 9, 10).

5. Vis implantable selon l'une quelconque des revendications précédentes, ***caractérisée en ce qu'** une* partie filetée (5, 9, 10) comporte un filetage osseux.

6. Vis implantable selon l'une quelconque des revendications précédentes, ***caractérisée en ce que*** le moment d'inertie de surface axial de la vis est inférieur de 30%, de préférence de 50% par rapport à une vis ayant le même diamètre extérieur.

7. Vis implantable selon l'une quelconque des revendications précédentes, ***caractérisé en ce que*** le matériau utilisé peut être du métal biocompatible ainsi que du plastique biocompatible résorbable ou non résorbable ou une combinaison de tels matériaux.
